# EUROPEAN PATENT SPECIFICATION

(11) **EP 2 043 524 B1**
(45) Date of publication and mention of the grant of the patent: **02.11.2016**
(21) Application number: 07720045.9
(22) Date of filing: 22.06.2007
(51) Int. Cl.: A61B 6/14, A61L 2/04, A61L 2/16

(54) **APPARATUS AND METHOD FOR DETECTING DENTAL PATHOLOGIES**
GERÄT UND VERFAHREN ZUM NACHWEIS VON ZAHNERKRANKUNGEN
APPAREIL ET PROCÉDÉ DE DÉTECTION DE PATHOLOGIES DENTAIRES

(30) Priority: 23.06.2006 US 805661 P
(43) Date of publication of application: 08.04.2009
(73) Proprietor: Dentsply Canada Ltd., Woodbridge, ON L4L 4A3 (CA); Karazivan, Naim, Repentigny, QC J5Y 3V7 (CA); Trottier, Sylvain, Saint-Lambert, QC J4S 1E3 (CA); Cyr, Claude, Longueuil, QC J4J 5K9 (CA); Gagnon, Stéphane, Laval, Québec H7L 5K3 (CA); Haynes, Francis, Terrebonne, QC J6X 2V9 (CA)
(72) Inventor: Karazivan, Naim, Repentigny, QC J5Y 3V7 (CA); Trottier, Sylvain, Saint-Lambert, QC J4S 1E3 (CA); Cyr, Claude, Longueuil, QC J4J 5K9 (CA); Gagnon, Stéphane, Laval, QC H7L 5K3 (CA); Haynes, Francis, Terrebonne, QC J6X 2V9 (CA)
(74) Representative: Reinhardt, Harry
(86) International application number: PCT/CA2007/001131
(87) International publication number: WO 2007/147262

(56) References cited:
- US-A- 5 040 539
- US-A- 5 124 797
- US-A- 5 570 182
- US-A1- 2003 053 061
- US-A1- 2005 019 722
- US-A1- 2005 255 424
- US-B1- 6 179 611

## Description

### FIELD OF THE INVENTION

The invention relates to devices for detecting dental pathology and more specifically to optical devices for detecting dental pathology.

### BACKGROUND OF THE INVENTION

There are various known methods that are used to detect the presence of dental caries, including visual and tactile investigations using the usual dental explorer. These methods and instruments have their limits and cannot detect dental caries reliably. X-ray investigation of teeth structure is also not reliable for detecting dental caries at the beginning of their formation in regions where a too great superimposition of enamel is present on the X-ray film. These obstructing superimpositions of teeth structures are more typical for the occlusal aspect of the teeth, and when the angle between the teeth alignment and the X-ray irradiation axis induces superimposition. The X-ray evaluation technique also exposes the patient to potentially harmful radiations.

Transillumination is another technique used to detect dental caries. By irradiating visible light toward a tooth from an aspect (e.g. lingual) and by observing via another aspect (e.g. buccal) the transmitted light, the operator can sometimes confirm the diagnosis of dental caries by observing a luminosity contrast induced by a dental caries. This technique is not suitable for all dental caries, especially for dental caries at their beginning phase. Recently, a viewing device has been developed to ease the viewing of transillumination of the teeth structure with the use of a camera.

Other devices have been devised for the detection of dental caries using luminescence or fluorescence spectroscopy with variable efficiencies depending, amongst others, on the cleanliness of the tooth surface. When irradiated with one or more initial radiations at a specific wavelength, some tooth structures generate a second radiation with a wavelength that is different from the initial radiations. The intensity and wavelength of such a second radiation is different for sound tooth structures from those for decayed tooth structures. See U.S. Pat. No. RE31,815, No. 4,479,499, No. 6,186,780, No. 6,102,704, No. 6,053,731, No. 6,135,774 and No. 5,306,144, and German Patent Publications No. DE-30 31 249-C2, No. DE-42 00 741-A1, No. DE-U1-93 17 984, No. DE-303 1249-C2 and No. DE-19541686-A1. However the fluorescence signal can be weak making detection difficult.

For instance, German Patent Publication No. DE-93 17 984-U discloses a device for the detection of dental caries using a light emission unit emitting pulsed light beams and a detection unit being sensitive during a time interval delayed with respect to the emitted light pulse.

German Patent Publication No. DE-42 00 741-A1 and European Patent Publication No. EP-0 555 645-B1 describe a device for the detection of dental caries via a radiation source, working in a wavelength range from 360-500 nanometers and detecting filtered reflected radiation of wavelengths between 620 and 720 nanometers with respect to the presence or absence of dental caries.

German Patent Publication No. DE-297 04 185-U is directed to a device for the detection of caries, plaque or bacterial infections of teeth comprising an emission/detection unit which has a plurality of individual emission fibers.

German Patent Publication No. DE-197 09 500-C1 teaches a method for the detection of dental caries, plaque or bacterial infections of teeth by comparing fluorescent light levels of different portions of a tooth in order to find those parts of the tooth which are most seriously affected by the caries.

German Patent Publication No. DE-297 05 934-U discloses a device for diagnosing tooth composition using a first light source as a detection light source and a second light source as a therapeutic light source.

Electrical probes have also been developed for the detection of dental caries (U.S. Pat. No. 6,230,050) as well as ultrasonic-based detection systems.

International patent application WO03/094771 discloses a system for detection of dental caries that relies on the detection of reflected/transmitted infrared light.

US-patent application US 2003/0053061 A1 which presents the closest prior art shows an instrument and a related process for measuring colour, shade, gloss, shape and/or translucence of a tooth. It provides a disposable protective shield over the areas that come into contact with the patient. This shield may be disposable or reusable, wherein in the case of reusable shields it is constructed of a material that can withstand sterilization in a typical autoclave, hot steam, chemiclave, or sterilizing system.

US-patent application 2005/255424 A1 shows a dental device for the investigation of the optical properties of tooth issue, wherein parts of the device are integrated in a dental handpiece. A sterile protect sleeve is added onto the distal end of the device that contacts the patient. The forward region of the handpiece is provided with a removable sleeve of sterilizable material. Solely the sleeve and the investigation probe come into contact with the patient and can be removed from the handpiece and separately sterilized. Electronic components of the device remain in the region of the handpiece which is not cleaned.

US-patent 5,124,797 A and US-patent application US 2005/0019722 A1 show cameras for viewing the inside of a mouth and are connected with wires/cables to external components. Thus, both systems comprise components, which are outside of the handheld device.

Despite the availability of the above systems there is a need for improved detectors of dental caries and other dental pathologies.

### SUMMARY OF THE INVENTION

In accordance with the present invention, a hand-held optical diagnostic tool for detection of dental pathologies is provided that functions as a self-contained unit. The diagnostic tool can detect pathologies such as caries, tartar, plaque and the like using an optical signal. The signal penetrates, at least partially, a tooth structure, and the reflected or transmitted optical energy is indicative of structural details within the tooth.

In an exemplary embodiment of the invention, the diagnostic tool has an optical probe and a housing within which an optical source, an optical detector, an analysis processor and a power source are located. The electrical components, namely the optical source, optical detector, analysis processor and power source, are integrated on a support which is removably inserted in the housing. To allow removal of the support, the housing has multiple portions, such as a proximal end and a distal end for a two-piece housing. The proximal and distal ends connect to each other with the support located inside to form a self-contained, handheld diagnostic tool.

The optical source and optical detector may be adapted to generate and detect light, respectively, at two or more distinct wavelengths. Each of the optical source and optical detector may also be made up of one or more discrete components, such as an LED for the optical source and a photodiode for the detector. The optical source couples an incident light beam of optical energy through one or more incident light ports, and the optical detector receives optical energy through one or more detection ports. In one variation, each of the incident light ports and detector ports may be dedicated to transmission or detection of a different wavelength range.

The light probe of the invention conducts light from the optical source to a tooth under examination, and conducts light from the tooth to the detector. The light probe may be connected to an adapter on a distal end of the housing, and may comprise optical fibers that transport the light. Different distal ends may also be used with the same proximal end, each being adapted to a different dental pathology. Thus, the distal ends may be changed for performing a different type of examination. In such a case, a unique identifier may be included with each distal end.

The incident light ports and detection ports may have predetermined numerical apertures which may be adapted to control an amount and shape of the incident light beam and the optical energy received by the detector. These ports may also be arranged so as to minimize overlapping between the incident light beam and the detected light. In one variation, an optically opaque divider may be provided between the incident light port and the detection port to prevent interaction between the incident light and the detected light. Optical couplers are provided to couple the light source and the optical probe. The couplers may include a male portion and a female portion that are engaged when the proximal and distal ends of the housing are connected together.

The proximal end and the distal end of the housing may be manually connected together and separated. The connection may be, for example, a pressure fit, or may use a manually displaceable detent means. The ability to separate the housing portions and remove the support with the electrical components simplifies the task of sterilizing the tool. With the more sensitive support components removed, the light probe and the housing portions may be placed in an autoclave, or may be chemically sterilized without risk to the tool.

The analysis processor also allows various functionality to the tool, including the ability to detect certain known pathologies and generate a signal indicative of their detection. The processor may also be used to initiate a "sleep" mode when the detected signal indicates that no examination is being performed. In this mode, power to the optical source and detector is reduced. As the power source is typically a battery, this reduces unnecessary power consumption.

### BRIEF DESCRIPTION OF THE DRAWINGS

Further features and advantages of the present invention will become apparent from the following detailed description, taken in combination with the appended drawings, in which:
Figure 1 is a perspective view of a dental pathology diagnostic tool according to the invention;
Figure 2 is a front view of a light probe of the diagnostic tool of Figure 1, showing an optically opaque divider at the tip of the probe;
Figure 3 is a graphical representation of reflected light intensities for a diagnostic tool like that shown in Figure 1;
Figure 4 is an exploded perspective view of the diagnostic tool of Figure 1; and
Figure 5 is a side view of the diagnostic tool of Figure 1 showing a proximal end, a support and a distal end.

### DETAILED DESCRIPTION OF THE INVENTION

In accordance with the present invention there is provided a diagnostic tool for detecting dental pathologies such as caries, residual caries, secondary caries, plaque, tartar and the like. The operating principle of the device is the measurement of the reflection of an incident beam of light that is directed at a tooth region.

The diagnostic tool is a hand-held device that generally comprises within a housing one or more light sources, one or more light detectors, a power supply and a processor for analyzing the detected light and generating an output indicative of the presence or absence of dental pathologies. The device is therefore self-contained.

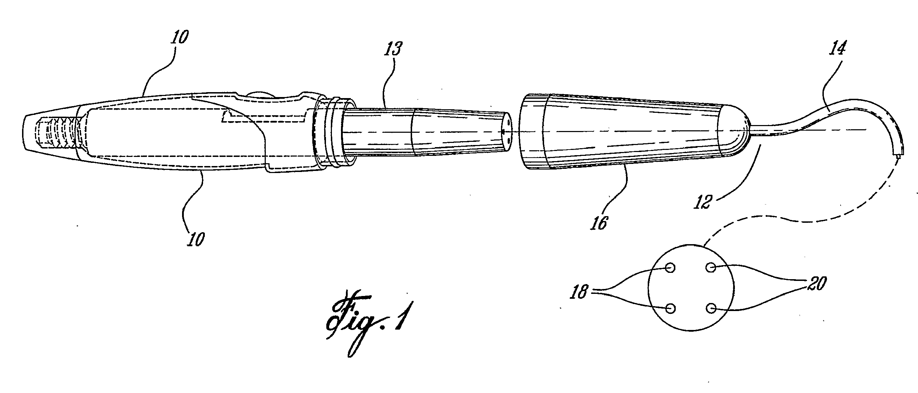

An exemplary embodiment of the diagnostic tool is shown in Figure 1 and includes a housing comprising a proximal end 10 and a distal end 12. The distal end comprises a light probe 14 and an adaptor 16, which can be removably connected to the distal end. The light probe 14 has incident light ports 18 and detector ports 20 through which the incident light from a light source exits and reflected/transmitted light is collected, respectively. Although the figure shows two incident light ports and two detector light ports, it will be appreciated that the light probe may comprise any number of incident light ports and detector ports.

As will be further explained below, light can be propagated within the device through known optical components such as mirrors or optical fibers. In the embodiment of Figure 1, optical fibers are used, and optical coupling between the different parts of the device uses known optical couplers. The invention may make use of an optical fiber or bundle of optical fibers similar, for example, to some of the optical fibers used for laser-based endodontic treatment. The optical coupling can also be provided by lens(es) and/or mirror(s) or any other material suitable for radiation transmission. It will be appreciated that filters can be used to select appropriate wavelengths or to filter out undesired wavelengths.

In the exemplary embodiment, optical coupling between two parts of the device is provided with optical shielding to prevent cross-talk between channels as for example by using plugs. In one variation, the core diameter of the fibers can be chosen such that the light receiving end of a first fiber is larger than the light exiting end of a second fiber to which the first fiber is coupled, thereby providing a greater cross-sectional area and ensuring that substantially all the light is captured and passed through the optical circuit.

The numerical apertures of the incident light port(s) and detection port(s) can be selected to control the amount of light that is injected on the surface of the tooth and that is detected after reflection. The depth of the region being probed can be increased by selecting appropriate numerical apertures to increase the amount of light being injected. However the numerical apertures of the incident light port and the detection port should be chosen so as to avoid overlap of the incident and detected light. In the embodiment of Figure 1, the numerical apertures are between 0.18 and 0.7.

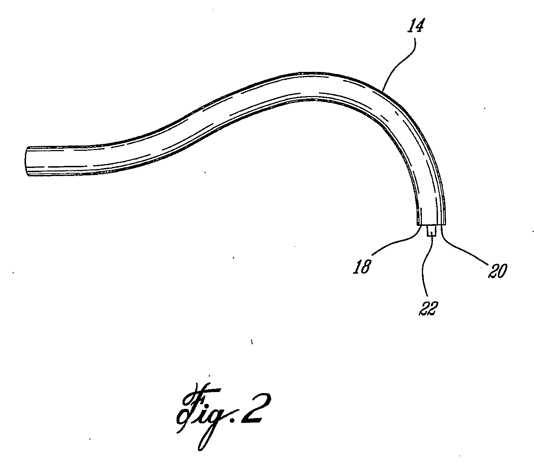

To avoid the problem of light interference between the incident and detected light an optically opaque divider 22 (Figure 2) can be placed at the end of the light probe between the incident light ports and the detection ports. This divider 22 may be a permanent piece affixed to the end of the light probe and may be, for example, molded plastic. The positioning of the divider 22 is such that the detection angle of the detection ports is limited in the direction of the divider, and such that any light emitted from the incident light ports at too sharp an angle is blocked by the divider.

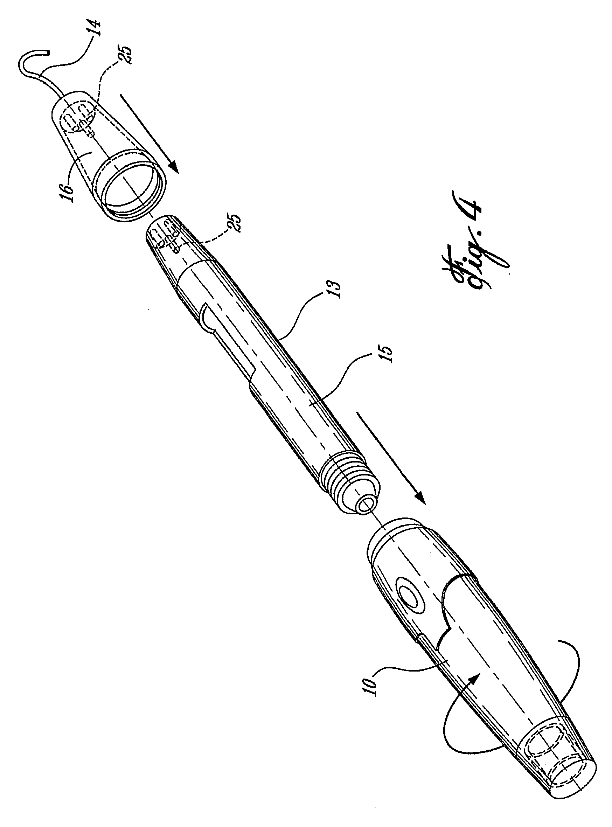

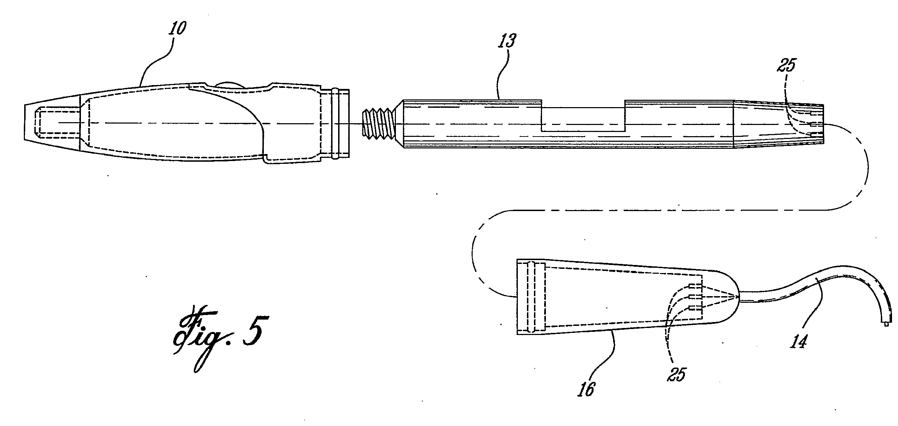

Referring back to figure 1, the electronic components used for controlling and analyzing light signals as well as the power source can be incorporated in a support 13 that can be removably inserted in the housing, preferably in the proximal end 10. The support 13 is a structure that can hold the desired components and, in the exemplary embodiment, it comprises a circuit board. The support 13 is made to be removable for ease of changing or sterilizing the housing, replacing parts and the like. Figures 4 and 5 show one favorable embodiment of the support 13. The electronic components are mounted to a circuit board that is located inside of a sleeve 15 that is shaped to fit within the proximal end 10 and the distal end 12 of the housing. The overall length of the sleeve is such that it may be completely contained by the two housing portions when they are connected together.

One particularly advantageous feature of the present invention is that all of the external surfaces may be sterilized without affecting the electrical components of the support 13. Thus, for example, the support 13 may be removed and the two housing portions 10, 12 placed in an autoclave. With all of the temperature sensitive elements of the tool being located with the support 13, the housing portions, along with the light probe 14, can thus be easily sterilized without damage to the tool. Moreover, as the support is completely contained within the housing portions, all surfaces that come in contact with the patient are steriziled. The sterilization of the housing (including the light probe) can also be effected by chemical sterilization, with the removable aspect of the support 13 providing the same advantages. In an alternative embodiment, the housing portions 10, 12 can be disposable. Since the more expensive components may be all located with support 13, this option may be desirable.

In the exemplary embodiment, the housing is detachable at the junction between the proximal and distal ends. As indicated above, the distal end comprises a light probe adapted to direct the light onto the surface of a tooth and to collect reflected/transmitted light. In the embodiment shown, the distal end comprises a probe having a generally curved shape which facilitates access to the teeth surfaces within the mouth so as to direct the incident light on a desired surface and to collect reflected light. The light probe 14 is attached and optically coupled to adaptor 16. The adaptor comprises optical components such as optical fibers and/or mirrors for directing the light, originating from the light source, to the probe and ultimately to the incident light ports. Likewise, the adaptor 16 has similar optical component for collecting the reflected/transmitted light and directing it to the optical detector.

As shown in Figures 4 and 5, optical connectors 25 are provided between tadaptor 16 and support 13. The optical connectors may be any type of known connector and, in the embodiment shown, are plug and socket type connectors. The male portions of the connectors 25 are located in the adaptor 16, while the female portions reside with the support 13. When the support 13 is inserted into the adaptor 16, the male portions engage the female portions and align the light-carrying elements of the support and the adaptor. By assuring proper optical alignment, the connectors ensure a low degree of optical signal loss for the optical signals passing through the incident light and detection ports.

The source of light for the diagnostic tool may also take different forms and, for the present embodiment, is an electromagnetic radiation generator that can generate light within desired wavelength ranges of the UV-Visible-IR spectrum. The light source may therefore be made up of multiple sources that operate in different wavelength ranges and are coupled together to provide the different desired wavelength contributions. Filter(s) or other optical means can also be used to obtain the desired wavelength ranges. For example, visible light of around 630 nm wavelength can be combined with infrared radiation of around 860 nm wavelength. Examples of discrete components that may make up the light source include LEDs, laser-diodes, lasers, halogen lights, neon lights, or any other suitable type of radiation-emitting source. LEDs are used in the present embodiment due to their relatively low power operation and compact size. It will be appreciated that, regardless of the specific source components, the light intensity should be adjusted so as to avoid causing harm to the patient.

The detector may be any suitable detector such as a semiconductor detector (e.g., photodiode or LCD), or a photoresistor.

In the exemplary embodiment, the detector and the light source are located with the support 13. This ensures that, if temperature-sensitive or chemically sensitive source and detector components are used, they will have been removed before any potentially destructive sterilization is applied to the housing components. However, those skilled in the art will also recognize that the source and detector components could be located in the adaptor 16. In such a case, the components would have to be adequately sealed against the possibility of thermal or chemical degradation during a corresponding sterilization procedure, or a more surface specific sterilization process should be used.

The proximal and distal ends of the diagnostic tool are detachably connected together so as to provide proper alignment of the parts and, in particular, alignment of the optical components. In this respect, the support 13 may comprise one or more light sources and one or more detectors that are optically coupled to the adaptor. As such, the housing of the proximal end and the support are adapted such as to properly align the support when it is inserted in the housing. Similarly the adaptor may be provided with proper connection guiding means to ensure proper connection to the support and the proximal end. As mentioned above, it is possible for the light source and/or the light detector to be located in the adaptor. In such a case, electrical coupling is provided between the support and the adaptor to carry electrical signals to and from the electrical circuitry located at the support.

In the present embodiment, a positive connection is provided between the proximal and distal ends. That is, the two ends fit together snugly to form an integral tool. The connection may be of different types, such as a pressure fit or a connection that uses a manually displaceable detent. The use of such a mechanism may also be such as to provide a signal to the user that the parts are properly connected. For example the distal end (through the adaptor) can be designed to "snap on" to the proximal end in a way that engages the detent and mechanism and correspondingly provides an audio and sensation signal to the user.

When a tooth structure is irradiated with an incident beam of light, the radiation is typically reflected in part from the structure surface and transmitted in part such that it penetrates the structure. Within the structure of the tooth, some or all of the penetrating radiation can be deviated and/or reflected. Depending of the composition and/or shape of the tooth, a specific wavelength may be reflected or transmitted differently than it would be when encountering a different structure. It will be appreciated that the intensity of the reflection also depends on the configuration of the injection and detection positions.

Thus the device relies on the measurement of the reflectance and/or transmittance properties of structures related to pathologies when these structures are irradiated with light of an appropriate wavelength such as visible or invisible ultra-violet (UV) or invisible infrared (IR) wavelength(s). The wavelength can be chosen as a function of the pathology. That is to say, the wavelength can provide specificity for the identification of the pathology. In this respect an analysis processor may be provided that automatically generates a signal that indicates the type of pathology that has been detected.

Light is brought to a region of a tooth by the light probe. In one embodiment of the invention the incident light is provided at two or more wavelengths. The reflected light is detected for each wavelength to provide measurements that are combined to derive values indicative of the presence of dental pathologies. For example, an infrared electromagnetic radiation of around 860 nm can be used alone or with an electromagnetic radiation of around 630 nm. Any other suitable wavelength or group of two or more wavelengths in the UV, visible or IR spectrum can be used.

It will also be appreciated that a visible wavelength can be used to provide visual guidance to the user at the same time as providing optical data for the detection of pathologies.

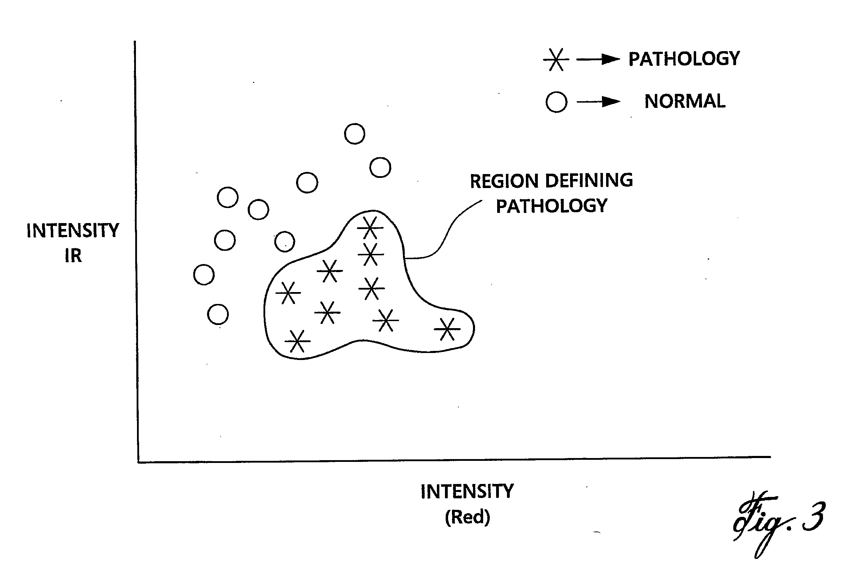

When a plot of the intensity of reflected light at one wavelength *vs.* the intensity of reflected light at another wavelength is obtained, regions of the plot correspond to a reflection characteristic of a state of the region of the tooth from which the reflection originates. Thus the relationship between the reflections at different wavelengths provides values indicative of healthy or damaged tooth tissue. A schematic representation of a plot of the intensity of reflected light at two wavelengths is shown in Figure 3.

The processor can respond to detected signals in any of a number of desired ways. Since different pathologies have varying effects on the selected wavelengths, the response parameters will depend on the specific application, the incident wavelengths, as well as other factors surrounding the diagnostic tool and procedure. However, the comparison of reflections using two different wavelengths is a known technique, and may be used with a configuration like that of the present embodiment. The data collected by the processor may be stored for subsequent examination. In addition, when the signals detected have the characteristics of a pathology of interest, the processor may identify these characteristics and generate a signal to alert the user. The signal may be an audible signal, a visual signal, a tactile signal or combination thereof.

In one embodiment of the invention, the processor can also generate a signal upon detecting reflected light that is not from a tooth surface or, if there is absence of reflected/transmitted light, to activate an auto shutdown mechanism. That is, if a detected signal indicates that the diagnostic tool is not being used for tooth analysis, the system would enter a "sleep" mode, in which energy for the power source is economized. Such sleep systems are known, particularly with regard to data processors, and the specifics of their implementation is not discussed in detail herein. In one embodiment, the sleep mode may include reducing the intensity of the incident light while there is no signal or while the detected signal is from a surface other than that of a tooth, and returning the light to its normal intensity when a tooth surface is detected. It is also possible that the device be completely turned off when no tooth surface is detected for a predetermined amount of time.

As mentioned, the diagnostic tool of the present embodiment is completely self-contained. As such, power is provided by one or more batteries. The battery or batteries can be removably inserted in the support 13. Alternatively, one or more rechargeable batteries may be permanently inserted in the support..

The probe and the adaptor of the device can be designed to detect a specific pathology. This can be achieved, for example, by providing the appropriate optical components (LEDs, filters etc.) to operate the device at one or more predetermined wavelengths. In addition, several interchangeable adaptors/probes (distal end) can be provided to provide different lengths or shapes as may be desired. In this context, the distal end may be provided with a proper identifier. However, it will also be appreciated that a single adaptor/probe may be used to detect multiple pathologies.

When multiple (two or more) wavelengths are used simultaneously the detection can be performed by modulating the incident beam of light and synchronizing the modulation with the detector to distinguish the reflected/transmitted light from the incident beam, from other sources of light and from noise. This method is sometimes called a "lock-in system". One advantage of the lock-in system is its sensitivity even with very weak levels of radiation. Alternatively, the different wavelengths can be discriminated by using an "on/off' mechanism by which one wavelength is used at a time with the other wavelength(s) being switched off. The modulation and "on/off" techniques may also be used together. Notwithstanding these distinctions, those skilled in the art will understand that the same detector may be used for detecting the different wavelengths.

The number of incident light ports 18 and detector ports 20 may be chosen according to a desired application. In the present embodiment, two incident light ports are shown along with two detection ports. Each port is dedicated to the emission or detection of a particular wavelength or range of wavelengths which may be achieved, for example, by using appropriate filters. This arrangement also enables the use of several wavelengths simultaneously without recourse to modulation or "on/off" mode. The system may also use just a single port for the detector, and the control of signal transmission may be used to keep the signals segregated. It may also be desirable, in such an instance, to use just a single incident light port, and to couple together the light from different sources, if more than one are used. The light from the different sources would thus share the same optical paths through the device.

To enhance the detection, it may be desirable to characterize the typical response radiation with regard to different regions (e.g., healthy tooth) in the mouth of some patients prior to beginning the examination of dental pathology. In such a way, a baseline signal can be established by obtaining measurements from the baseline region, such as from healthy tissue. It will be appreciated that the baseline signal can also be established under "laboratory" conditions and stored in a memory within the electronic components of the device.

The device of the present invention can also include some recalibration and/or self-testing functions. For example, if optical fibers are used, it is possible to verify if the fibers are too worn out to be efficiently used and should thus be replaced by testing the intensity of a reference light that is passed through the fibers. Other self-testing functions may include the verification of battery levels.

The analysis processor of the device may comprise or be connected to a programmed microprocessor or microcontroller, such as a digital signal processor (DSP) and peripheral integrated circuit elements.

In particular processors such as DSPs provides flexibility to the device by enabling a variety of functionalities. For example, as the diagnostic tool may have the ability to detect which distal end is connected to the device. The unit may also have a "sleep" mode in which the power consumption is reduced. In this mode, the DSP provides a pulse at regular intervals and, from the reflected light characteristics, determine whether the light is being directed at a tooth. the diagnostic tool may also have the ability to store data of an analysis so that it can be reviewed later at a workstation. Other possible variations include the ability to wirelessly communicate with a base station using a wireless transceiver controlled by the DSP, the ability to change the analysis algorithm by upload to the device from a base station, and the like.

For performing data transfer, the present embodiment of the diagnostic tool includes the ability to connnect to a network by any available means. Such means may include a direct cable connection, wireless connection, a connection over a wide area network or a local area network, a connection over an intranet, a connection over the Internet, or a connection over any other distributed processing network or system. The invention can also comprise an archiving feature in which collected data is saved for later use, for example, in a computer that may be connected to the tool.

In the present embodiment, the size shape and form figure of the diagnostic tool are made to be ergonomic for the dental professional and the patient. In addition, the device is sized such that it fits in station used by dentists, for example, in a standard dental tool holder. In this way, the tool may be simply added to an existing group of dental tools without requiring any extensive modification.

The diagnostic tool of the present invention may be used for treating an individual in need thereof by enabling a user to detect a pathology. An optical tool such as this is particularly useful for the detection of dental caries, tartar, plaque and the like. Once diagnosed, the dental professional may apply an appropriate treatment such as removing the decayed tooth matter or tartar and filling the tooth when the pathology is caries. During such a procedure, the also invention may be used to verify if the decayed matter or the tartar has been completely removed.

The invention may also be adapted to work with a three-dimensional (3D) dental map. In such a case, the tool can register the detection of a signal indicating the presence of a pathology with the three-dimensional location of the pathology. This can be achieved for example by registering the device with the dental map and displaying said map together with an indication of the location of the pathology.

While the invention has been described in connection with specific embodiments thereof, it will be understood that it is capable of further modifications and this application is intended to cover any variations, uses, or adaptations of the invention following, in general, the principles of the invention and including such departures from the present disclosures as come within known or customary practice within the art to which the invention pertains and as may be applied to the essential features herein before set forth, and as follows in the scope of the appended claims.

## Claims

1. A dental pathology diagnostic tool for examining the structure of a tooth, the tool comprising:
a housing comprising a proximal portion (10) and a distal portion (12) removably coupled to one another;
an optical source for generating optical energy in a predetermined wavelength range;
an optical detector for detecting optical energy in a predetermined wavelength range and generating a signal indicative thereof;
an optical probe (14) for conducting optical energy from a light source to the tooth and for conducting optical energy from the tooth to the light detector, said probe being autoclavable;
an analysis processor that receives the detector signal and analyzes it relative to predetermined signal characteristics indicative of dental pathology; and
a power source that provides power to each of the optical source, optical detector and analysis processor, wherein, during operation, the optical source, the optical detector, the analysis processor and the power source are located within the housing;
**characterized in that** the two housing portions are autoclavable and **in that** the optical source, the optical detector, the analysis processor and the power source are mounted to a circuit board that is located inside of a sleeve (15) that is shaped to fit within the proximate portion and the distal portion of the housing, the overall length of the sleeve being such that it may completely be contained by the two housing portions when they are connected together to form a self-contained, handheld unit.

2. The diagnostic tool as claimed in claim 1 wherein said optical probe (14) and distal portion (12) of said housing comprise an optical coupler (25) inside said distal portion of said housing for coupling said optical energy between said probe and said source and said detector, said optical probe and said optical coupler (25) being autoclavable with said distal portion.

3. The diagnostic tool as claimed in claim 1 wherein said optical source and optical detector are adapted to provide and detect light at two or more wavelengths.

4. The diagnostic tool as claimed in claim 1 wherein said optical source couples an incident light beam of optical energy through one or more incident light ports (18) and wherein said optical detector receives optical energy through one or more detection ports (20), wherein, in the case of a plurality of incident light ports and/or detection ports, preferably each port is dedicated to transmission or detection at a different wavelength range.

5. The diagnostic tool as claimed in claim 1 wherein said optical source comprises a light emitting diode (LED) and/or wherein said detector comprises a photodiode.

6. The diagnostic tool as claimed in anyone of claims 1-5 wherein the distal portion (12) is a first distal portion and wherein the diagnostic tool comprises a plurality of interchangeable distal portions each adapted for detection of a different predetermined dental pathology, the diagnostic tool preferably further comprising an identifier for each distal end.

7. The diagnostic tool as claimed in claim 4 wherein said incident light ports (18) and detection ports (20) have predetermined numerical apertures and wherein said apertures are adapted to control an amount and shape of the incident light beam and the optical energy received by the detector,

8. The diagnostic tool as claimed in claim 7 wherein the incident light ports (18) and the detection ports (20) are arranged so as to minimize overlapping between said incident light beam and said detected light and/or wherein said numerical apertures are between 0.18 and 0.7.

9. The diagnostic tool as claimed in claim 2 wherein said optical coupler (25) comprises an incident light port and a detection port separated by an optically opaque divider (22).

10. The diagnostic tool as claimed in claim 1 wherein said distal portion (12) and said proximal portion (10) are adapted to generate a signal when connected together to indicate to a user that they are properly connected.

11. The diagnostic tool as claimed in claim 1 wherein said housing is disposable.

12. The diagnostic tool as claimed in claim 1 wherein said light probe (14) is **characterized by** a substantially curved shape to facilitate access to teeth surfaces within a mouth.

13. The diagnostic tool as claimed in claim 1 wherein said diagnostic tool is adapted to automatically detect a type of dental pathology and to provide an indication of its detection.

14. The diagnostic tool as claimed in claim 2 wherein said analysis processor controls power transfer from the power source to one or both of the optical source and optical detector, and wherein the analysis processor initiates a "sleep" mode in which power transfer from the power source to one or both of the optical source and optical detector is reduced when light is detected from a surface other than a tooth surface or when there is absence of detected signal, wherein the "sleep" mode preferably includes a reduction in activity by the analysis processor to further conserve energy.

15. The diagnostic tool as claimed in claims 14 wherein said power transfer from the power source to one or both of the optical source and optical detector is automatically returned to normal when tooth surface is detected.

16. The diagnostic tool as claimed in claim 14 wherein said analysis processor can provide a signal to turn the diagnostic tool off after a predetermined amount of time without tooth surface signal detection.

17. The diagnostic tool as claimed in claim 13 wherein a signal indicative of presence of a dental pathology is selected from the group consisting of a sound signal, a light signal and a vibration signal.

18. The diagnostic tool as claimed in claim 1 wherein said power source is a battery that is replaceably inserted in said support or a rechargeable battery that is permanently inserted in said support.

19. A method for maintaining a dental pathology diagnostic tool for examining the structure of a tooth, wherein the diagnostic tool comprises
a housing having a distal portion (12) and a proximal portion (10),
an optical source for generating optical energy in a predetermined wavelength range, an optical detector for detecting optical energy in a predetermined wavelength range and generating a signal indicative thereof,
an optical probe (14) for conducting optical energy from a light source to the tooth and for conducting optical energy from the tooth to the light detector, said probe being autoclavable,
an analysis processor that receives the detector signal and analyzes it relative to predetermined signal characteristics indicative of dental pathology. and
a power source that provides power to each of the optical source, optical detector and analysis processor, and
wherein said optical source, optical detector, analysis processor and power source are manually removable from the housing and, during operation, the optical source, the optical detector, the analysis processor and the power source are located within the housing;
the method comprising:
removing the optical source, optical detector, analysis processor and power source from the housing;
sterilizing the two housing portions and the optical probe (14);
**characterized by** the step of connecting the optical source, the optical detector, the analysis processor and the power source to a circuit board that is located inside of a sleeve (15) that is shaped to fit within the proximate portion and the distal portion of the housing, the overall length of the sleeve being such that it may completely be contained by the two housing portions when they are connected together, and joining the two housing portions together to form a self-contained, handheld unit.

20. The method as claimed in claim 19 further comprising a support (13) with which the optical source, optical detector, analysis processor and power source are integrated, the support being removably locatable within the proximal housing portion (10).

21. The method as claimed in any of claims 19-20 wherein the sterilizing comprises sterilizing in an autoclave or chemical sterilizing.

22. The method as claimed in claim 19 wherein said plurality of sections are held together by a pressure fit or by one or more manually displaceable detents.

## Patentansprüche

1. Dentalpathologisches Diagnoseinstrument zum Prüfen der Struktur eines Zahns, wobei das Instrument aufweist:
ein Gehäuse mit einem proximalen Abschnitt (10) und einem distalen Abschnitt (12), die lösbar miteinander gekoppelt sind;
eine optische Quelle zur Erzeugung optische Energie in einem vorbestimmten Wellenlängenbereich,
einen optischen Detektor zum Detektieren einer optischen Energie in einem vorbestimmten Wellenlängenbereich und zur Erzeugung einer diese indizierenden Signals;
eine optischen Sonde (14) zum Leiten optischer Energie von einer Lichtquelle zum Zahn und zum Leiten optischer Energie vom Zahn zu dem Lichtdetektor, wobei die Sonde autoklavierbar ist;
einen Analyseprozessor, der das Detektorsignal empfängt und es relativ zu vorbestimmten Signalcharakteristiken analysiert, die für einen dentalen pathologischen Befund indiziert sind; und
eine Energiequelle, die jedem der Elemente umfassend die optische Quelle, optischen Detektor und Analyseprozessor Energie zur Verfügung stellt, wobei während einer Operation die optische Quelle, der optische Detektor, der Analyseprozessor und die Energiequelle innerhalb des Gehäuses angeordnet sind;
**dadurch gekennzeichnet, dass** die beiden Gehäuseabschnitte autoklavierbar sind und dass die optische Quelle, der optische Detektor, der Analyseprozessor und die Energiequelle auf einer Leiterplatte montiert sind, die innerhalb einer Hülse (15) angeordnet ist, die so geformt ist, dass sie in den proximalen Abschnitt und den distalen Abschnitt des Gehäuses passt, wobei die Gesamtlänge der Hülse so ist, dass sie vollständig von den beiden Gehäuseabschnitten umfasst werden kann, wenn sie miteinander verbunden sind, um ein eigenständiges Handgerät zu bilden.

2. Diagnoseinstrument nach Anspruch 1, wobei die optische Sonde (14) und ein distaler Abschnitt (12) des Gehäuses einen Optokoppler (25) innerhalb des distalen Abschnitts des Gehäuses zum Koppeln der optischen Energie zwischen der Sonde und der Quelle und dem Detektor aufweist, wobei die optische Sonde und der Optokoppler (25) mit dem distalen Abschnitt autoklavierbar sind.

3. Diagnoseinstrument nach Anspruch 1, wobei die optische Quelle und optischer Detektor geeignet sind, Licht mit zwei oder mehr Wellenlängen zur Verfügung zu stellen und zu detektieren.

4. Diagnoseinstrument nach Anspruch 1, wobei die optische Quelle einen einfallenden Lichtstrahl optischer Energie durch ein oder mehr Lichteinfallports (15) koppelt und wobei der optische Detektor optische Energie durch ein oder mehr Detektionsports (20) empfängt, wobei im Fall einer Vielzahl von Lichteinfallports und/oder Detektionsports vorzugsweise jeder Port der Übertragung oder Detektion in einem unterschiedlichen Wellenlängenbereich gewidmet ist.

5. Diagnoseinstrument nach Anspruch 1, wobei die optische Quelle eine Leuchtdiode (LED) und/oder wobei der Detektor eine Fotodiode aufweist.

6. Diagnoseinstrument nach einem der Ansprüche 1 bis 5, wobei der distale Abschnitt (12) ein erster distaler Abschnitt vor, wobei das Diagnoseinstrument eine Vielzahl von untereinander austauschbaren distalen Abschnitten umfasst, von denen jeder zur Detektion eines unterschiedlichen vorbestimmten dentalen pathologischen Befunds geeignet ist, wobei das Diagnoseinstrument vorzugsweise ferner einen Identifizierer für jedes distale Ende aufweist.

7. Diagnoseinstrument nach Anspruch 4, wobei die Lichteinfallports (18) und Detektionsports (20) vorbestimmte numerische Öffnungen haben und wobei die Öffnungen geeignet sind, eine Menge und Form eines einfallenden Lichtstrahls und die vom Detektor empfangene optische Energie zu steuern.

8. Diagnoseinstrument nach Anspruch 7, wobei die Lichteinfallports (18) und die Detektionsports (20) angeordnet sind, um ein Überlappen des einfallenden Lichtstrahls und des detektierten Lichts zu minimieren und/oder wobei die numerischen Öffnungen zwischen 0,18 und 0,7 sind.

9. Diagnoseinstrument nach Anspruch 2, wobei der Optokoppler (25) einen Lichteinfallsport und einen Detektionsport aufweist, die durch einen optisch opaken Teiler (22) separiert sind.

10. Diagnoseinstrument nach Anspruch 1, wobei der distale Abschnitt (12) und der proximale Abschnitt (10) geeignet sind, ein Signal zu generieren, wenn sie miteinander verbunden sind, um einem Benutzer anzuzeigen, dass sie ordnungsgemäß verbunden sind.

11. Diagnoseinstrument nach Anspruch 1, wobei das Gehäuse disponibel oder ein Einwegartikel ist.

12. Diagnoseinstrument nach Anspruch 1, wobei die Lichtsonde (14) durch eine im Wesentlichen gekrümmte Form gekennzeichnet ist, um einen Zugang zu den Zahnoberflächen innerhalb eines Mundes zu erleichtern.

13. Diagnoseinstrument nach Anspruch 1, wobei das Diagnoseinstrument geeignet ist, selbsttätig einen Typ eines dentalen pathologischen Befundes zu detektieren und eine Anzeige seiner Detektion bereitzustellen.

14. Diagnoseinstrument nach Anspruch 2, wobei der Analyseprozessor die Energieübertragung von der Energiequelle zu einem oder beiden umfassend die optische Quelle und den optischen Detektor steuert und wobei der Analyseprozessor einen "Steep-Modus" initiiert, in dem eine Energieübertragung von der Energiequelle zu einem oder beiden umfassend die optische Quelle und den optischen Detektor reduziert ist, wenn Licht von einer anderen Oberfläche als einer Zahnoberfläche detektiert wird oder wenn es kein detektiertes Signal gibt, wobei der "Sleep-Modus" vorzugsweise eine Reduktion in der Aktivität durch den Analyseprozessor umfasst, um weiter Energie zu sparen.

15. Diagnoseinstrument nach Anspruch 14, wobei die Energieübertragung von der Energiequelle zur optischen Quelle und/oder zum optischen Detektor selbsttätig zum normalen Zustand zurückkehrt, wenn eine Zahloberfläche detektiert wird.

16. Diagnoseinstrument nach Anspruch 14, wobei der Analyseprozessor ein Signal bereitstellen kann, um das Diagnoseinstrument nach einer vorbestimmten Zeitspanne ohne Signaldetektion von einer Zahnoberfläche abzuschalten.

17. Diagnoseinstrument nach Anspruch 13, wobei ein Signal, das das Vorhandensein eines dentalen pathologischen Befundes indiziert, ausgewählt ist aus einer Gruppe umfassend ein Tonsignal, ein Lichtsignal und ein Vibrationssignal.

18. Diagnoseinstrument nach Anspruch 1, wobei die Energiequelle eine Batterie ist, die austauschbar in einem Träger eingesetzt ist, oder eine wiederaufladbare Batterie, die permanent in dem Träger eingesetzt ist.

19. Verfahren zum Warten eines dentalen pathologischen Diagnoseinstruments zum Prüfen der Struktur eines Zahns, wobei das Diagnoseinstrument aufweist
ein Gehäuse mit einem distalen Abschnitt (12) und einem proximalen Abschnitt (10),
eine optische Quelle zur Erzeugung einer optischen Energie in einem vorbestimmten Wellenlängenbereich, einen optischen Detektor zum Detektieren einer optischen Energie in einem vorbestimmen Wellenlängenbereich und zum Erzeugen eine diese indizierenden Signals,
eine optischen Sonde (14) zum Leiten optischer Energie von einer Lichtquelle zum Zahn und zum Leiten einer optischen Energie vom Zahn zu dem Lichtdetektor, wobei die Sonde autoklavierbar ist,
einen Analyseprozessor, der das Detektorsignal empfängt und es relativ zu vorbestimmten Signaleigenschaften analysiert, die einen dentalen pathologischen Befund indizieren, und
eine Energiequelle, die eine Energie an jedes der Elemente umfassend die optische Quelle, optischen Detektor und Analyseprozessor bereitstellt, und
wobei die optische Quelle, optischer Detektor, Analyseprozessor und Energiequelle manuell vom Gehäuse entfernbar sind und während einer Operation die optische Quelle, der optische Detektor, der Analyseprozessor und die Energiequelle innerhalb des Gehäuses angeordnet sind;
wobei das Verfahren aufweist:
Entfernen der optischen Quelle, optischen Detektors, Analyseprozessors und Energiequelle vom Gehäuse,
Sterilisieren der beiden Gehäuseabschnitte und der optischen Sonde (14);
**gekennzeichnet durch** den Schritt eines Verbindens der optischen Quelle, des optischen Detektors, des Analyseprozessors und der Energiequelle mit einer Leitplatte, die innerhalb einer Hülse (15) angeordnet ist, die so geformt ist, dass sie in den proximalen Abschnitt und den distalen Abschnitt des Gehäuses passt, wobei die Gesamtlänge der Hülse so ist, dass sie vollständig von den beiden Gehäuseabschnitten umfasst ist, wenn sie miteinander verbunden sind, und Verbinden der beiden Gehäuseabschnitte miteinander, um ein eigenständiges Handgerät zu bilden.

20. Verfahren nach Anspruch 19, wobei es ferner einen Träger (13) umfasst, mit dem die optische Quelle, optischer Detektor, Analyseprozessor und Energiequelle integriert werden, wobei der Träger lösbar in dem proximalen Gehäuseabschnitt (10) anordenbar ist.

21. Verfahren nach einem der Ansprüche 19 bis 20, wobei das Sterilisieren ein Sterilisieren in einem Autoklaven oder ein chemisches Sterilisieren umfasst.

22. Verfahren nach Anspruch 19, wobei eine Vielzahl von Abschnitten zusammengehalten werden durch eine Druckbefestigung oder Druckpassung oder durch ein oder mehrere manuell verschiebbare Rastungen.

## Revendications

1. Un outil de diagnostic de pathologies dentaires pour l'examen de la structure d'une dent, cet outil comprenant :
un boîtier comprenant une partie proximale (10) et une partie distale (12) couplées de manière amovible l'une à l'autre ;
une source optique pour générer de l'énergie optique dans une plage de longueurs d'ondes prédéterminée ;
un détecteur optique pour détecter de l'énergie optique dans une plage de longueurs d'ondes prédéterminée et générer un signal indicatif de cette énergie ;
un conduit optique (14) pour conduire de l'énergie optique d'une source de lumière à la dent et pour conduire de l'énergie optique de la dent au détecteur de lumière, ledit conduit étant autoclavable ;
un processeur d'analyse qui reçoit le signal du détecteur et l'analyse quant à des caractéristiques de signal prédéterminées indicatives d'une pathologie dentaire ; et
une source d'alimentation qui fournit de l'énergie à la fois à la source optique, au détecteur optique et au processeur d'analyse,
dans lequel, durant l'opération, la source optique, le détecteur optique, le processeur d'analyse et la source d'alimentation sont situés dans le boîtier ;
**caractérisé en ce que** les deux parties de boîtier sont autoclavables et **en ce que** la source optique, le détecteur optique, le processeur d'analyse et la source d'alimentation sont montés sur un circuit imprimé qui situé dans un manchon (15) qui est conformé pour s'insérer dans la partie proximale et la partie distale du boîtier, la longueur totale du manchon étant telle qu'il peut être contenu complètement dans les deux parties de boîtier lorsque celles-ci sont connectées ensemble pour former une unité autonome et portable.

2. L'outil de diagnostic selon la revendication 1, dans lequel lesdits conduit optique (14) et partie distale (12) dudit boîtier comprennent un coupleur optique (25) à l'intérieur de ladite partie distale dudit boîtier pour relier ladite énergie optique dudit conduit à ladite source et audit détecteur, ledit conduit optique et ledit coupleur optique (25) étant autoclavables avec ladite partie distale.

3. L'outil de diagnostic selon la revendication 1, dans lequel lesdits source optique et détecteur optique sont adaptés pour fournir et détecter de la lumière de deux ou plus longueurs d'ondes.

4. L'outil de diagnostic selon la revendication 1, dans lequel ladite source optique couple un faisceau lumineux incident d'énergie optique à travers un ou plusieurs ports de lumière incidente (18) et dans lequel ledit détecteur optique reçoit de l'énergie optique à travers un ou plusieurs ports de détection (20), dans lequel, dans le cas d'une pluralité de ports de lumière incidente et/ou de ports de détection, de préférence chaque port est dédié à la transmission ou détection à une longueur d'ondes différente.

5. L'outil de diagnostic selon la revendication 1, dans lequel ladite source optique comprend une diode électroluminescente (LED) et/ou dans lequel ledit détecteur comprend une photodiode.

6. L'outil de diagnostic selon l'une quelconque des revendications 1 à 5, dans lequel la partie distale (12) est une première partie distale et dans lequel l'outil de diagnostic comprend une pluralité de parties distales interchangeables, chacune étant apte à la détection d'une pathologie dentaire prédéterminée différente, l'outil de diagnostic comprenant de préférence en outre un identificateur pour chaque extrémité distale.

7. L'outil de diagnostic selon la revendication 4, dans lequel lesdits ports de lumière incidente (18) et ports de détection (20) ont des ouvertures numériques prédéterminées et dans lequel lesdites ouvertures sont aptes à contrôler une quantité et une forme du faisceau de lumière incidente et l'énergie optique reçue par le détecteur.

8. L'outil de diagnostic selon la revendication 7, dans lequel les ports de lumière incidente (18) et les ports de détection (20) sont agencées de manière à minimiser le recouvrement entre ledit faisceau de lumière incidente et ladite lumière détectée et/ou dans lequel lesdites ouvertures numériques sont comprises entre 0,18 et 0,7.

9. L'outil de diagnostic selon la revendication 2, dans lequel ledit coupleur optique (25) comprend un port de lumière incidente et un port de détection séparés par un diviseur optiquement opaque (22).

10. L'outil de diagnostic selon la revendication 1, dans lequel ladite partie distale (12) et ladite partie proximale (10) sont aptes à générer un signal lorsqu'elles sont connectées ensemble pour indiquer à un utilisateur qu'elles correctement connectées.

11. L'outil de diagnostic selon la revendication 1, dans lequel ledit boîtier est jetable.

12. L'outil de diagnostic selon la revendication 1, dans lequel ledit conduit optique (14) est **caractérisé par** une forme sensiblement incurvée pour faciliter l'accès aux surfaces des dents à l'intérieur d'une bouche.

13. L'outil de diagnostic selon la revendication 1, dans lequel ledit outil de diagnostic est apte à détecter automatique un type de pathologie dentaire et à fournir une indication de sa détection.

14. L'outil de diagnostic selon la revendication 2, dans lequel ledit processeur d'analyse commande le transfert d'énergie de la source d'alimentation à la source optique et/ou au détecteur optique, et dans lequel le processeur d'analyse démarre un mode « sommeil » dans lequel le transfert d'énergie de la source d'alimentation à la source optique et/ou au détecteur optique est réduit lorsque de la lumière est détectée depuis une surface autre qu'une surface de dent ou lorsqu'il n'y a pas de détection de signal, dans lequel le mode « sommeil » inclut de préférence une réduction de l'activité du processeur d'analyse pour conserver davantage d'énergie.

15. L'outil de diagnostic selon la revendication 14 dans lequel ledit transfert d'énergie de la source d'alimentation à la source optique et/ou au détecteur optique revient automatiquement à la normale lorsqu'une surface de dent est détectée.

16. L'outil de diagnostic selon la revendication 14, dans lequel ledit processeur d'analyse peut fournir un signal pour éteindre l'outil de diagnostic après un temps prédéterminé sans détection de signal de surface de dent.

17. L'outil de diagnostic selon la revendication 13, dans lequel un signal indicatif de la présence d'une pathologie dentaire est choisi dans le groupe constitué par un signal sonore, un signal lumineux et un signal vibratoire.

18. L'outil de diagnostic selon la revendication 1, dans lequel ladite source d'alimentation est une pile qui est insérée dans ledit support et peut être remplacée ou une batterie rechargeable qui est insérée de manière permanente dans ledit support.

19. Un procédé de maintien d'un outil de diagnostic de pathologie dentaire pour examiner la structure d'une dent, dans lequel l'outil de diagnostic comprend
un boîtier ayant une partie distale (12) et une partie proximale (10),
une source optique pour générer de l'énergie optique dans une place de longueurs d'ondes prédéterminée, un détecteur optique pour détecter de l'énergie optique dans une plage de longueurs d'onde prédéterminée et générer un signal indicatif de cette énergie,
un conduit optique (14) pour conduire de l'énergie optique d'une source lumineuse à la dent et pour conduire de l'énergie optique de la dent au détecteur de lumière, ledit conduit étant autoclavable,
un processeur d'analyse qui reçoit le signal de détection et l'analyse quant à des caractéristiques de signal prédéterminées indicatives d'une pathologie dentaire ; et
une source d'alimentation qui fournit de l'énergie à la fois à la source optique, au détecteur optique et au processeur d'analyse, et
dans lequel lesdits source optique, détecteur optique, processeur d'analyse et source d'alimentation peuvent être retirés à la main du boîtier et, durant le fonctionnement, la source optique, le détecteur optique, le processeur d'analyse et la source d'alimentation sont situés dans le boîtier ;
ce procédé comprenant :
le retrait des source optique, détecteur optique, processeur d'analyse et source d'alimentation du boîtier :
la stérilisation des deux parties de boîtier et du conduit optique (14) ;
**caractérisé par** l'étape consistant à connecter la source optique, le détecteur optique, le processeur d'analyse et la source d'alimentation à un circuit imprimé qui est situé à l'intérieur d'un manchon (15) qui est conformé pour s'insérer dans la partie proximale et la partie distale du boîtier, la longueur totale du manchon étant telle qu'il peut être contenu complètement dans les deux parties de boîtier lorsqu'elles dont connectées ensemble, et à assembler les deux parties de boîtier pour former une unité autonome et portable.

20. Le procédé selon la revendication 19, comprenant en outre un support (13) avec lequel les source optique, détecteur optique, processeur d'analyse et source d'alimentation sont intégrés, le support pouvant être disposé de manière amovible dans la partie de boîtier proximale (10).

21. Le procédé selon l'une quelconque des revendications 19 à 20, dans lequel la stérilisation comprend la stérilisation dans un autoclave ou une stérilisation chimique.

22. Le procédé selon la revendication 19, dans lequel ladite pluralité de sections sont maintenues ensemble par encliquetage ou par un ou plusieurs crans.
